(19) Europäisches Patentamt
European Patent Office
Office européen des brevets

(11) EP 1 264 589 A1

(12) **DEMANDE DE BREVET EUROPEEN**

(43) Date de publication:
**11.12.2002 Bulletin 2002/50**

(51) Int Cl.[7]: **A61K 7/027**, A61K 7/00, A61K 7/48

(21) Numéro de dépôt: **02291423.8**

(22) Date de dépôt: **07.06.2002**

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **07.06.2001 FR 0107474**

(71) Demandeur: **L'OREAL**
**75008 Paris (FR)**

(72) Inventeurs:
- **Livoreil, Aude**
  **75006 Paris (FR)**
- **Baghdadli, Nawel**
  **91300 Massy (FR)**

(74) Mandataire: **Catherine, Alain et al**
**Cabinet Harlé & Phélip**
**7, rue de Madrid**
**75008 Paris (FR)**

(54) **Utilisation d'un additif polaire dans une composition cosmétique comprenant une phase grasse liquide structurée par au moins un organogelateur pour donner à la composition un caractére thixotrope**

(57) L'invention concerne l'utilisation d'au moins un additif polaire ayant un paramètre de polarité $\delta_a$ égal ou supérieur à 7,0 $(J/cm^3)^{1/2}$, dans une composition cosmétique comprenant une phase grasse liquide contenant au moins une huile apolaire ou faiblement polaire ayant un paramètre de polarité $\delta_a$ inférieur à 7,0 $(J/cm^3)^{1/2}$, structurée par au moins un organogélateur moléculaire, pour conférer un caractère thixotrope à la composition.



Printed by Jouve, 75001 PARIS (FR)

## Description

La présente invention se rapporte à l'utilisation d'un additif polaire, dans une composition cosmétique de soin et/ou de maquillage de la peau et/ou des lèvres et/ou des phanères des êtres humains, comprenant une phase grasse liquide contenant au moins une huile apolaire ou faiblement polaire, structurée par au moins un organogélateur, pour conférer un caractère thixotrope à la composition.

Ces compositions, qui peuvent en particulier se présenter sous forme coulée notamment en sticks, conduisent après application à des dépôts non migrants, ne fracturant pas, non granuleux et faciles à étaler.

Par additif polaire, on entend, au sens de l'invention, un composé polaire caractérisé par un paramètre de polarité $\delta_a$ égal ou supérieur à 7,0 $(J/cm^3)^{1/2}$.

Par huile apolaire ou faiblement polaire, on entend au sens de l'invention une huile ayant un paramètre de polarité $\delta_a$ inférieur à 7,0 $J/cm^3)^{1/2}$.

Par paramètre de polarité, on entend, au sens de l'invention, le paramètre moyen $\delta_a$ traduisant la polarité d'une molécule : plus la valeur de $\delta_a$ est élevée, plus la polarité de la molécule est élevée.

Le paramètre moyen $\delta_a$ est défini en fonction des paramètres de solubilité de Hansen $\delta_p$ et $\delta_h$, suivant la relation suivante :

$$\delta_a = \sqrt{(\delta_p{}^2 + \delta_h{}^2)}$$

Les paramètres $\delta_p$ et $\delta_h$ caractérisent respectivement les forces d'interaction de Debye entre dipoles permanent et la capacité d'un composé à donner des liaisons hydrogène. Ces paramètres sont définis selon l'espace des paramètres de solubilité de HANSEN dans le document J. Paint Technology 39, 195 (1967) *"The Three Dimensional Solubility Parameter - Key to Paint Component Affinities".*

Par phase grasse liquide, on entend, au sens de l'invention, une phase grasse liquide à la température ambiante (25°C) et à la pression atmosphérique (76 mm de Hg), composée d'un ou plusieurs corps gras liquides à température ambiante, également appelés huiles, généralement compatibles entre eux.

Par corps gras liquide, on entend, au sens de l'invention, un milieu liquide non aqueux, non miscible en toute proportion à de l'eau.

Par organogélateur, on entend, au sens de l'invention, un composé organique non polymérique, moléculaire, dont les molécules sont capables d'établir entre elles des interactions physiques conduisant à une auto-agrégation des molécules avec formation d'un réseau supra-moléculaire D3 qui est responsable de la gélification de la phase grasse liquide.

Par gélification, on entend, au sens de l'invention, un épaississement du milieu pouvant conduire à une consistance gélatineuse et même à une consistance solide, rigide, ne s'écoulant pas sous propre poids.

Par composition ayant un caractère thixotrope, ou composition thixotrope, on entend, au sens de l'invention, une composition structurée qui fluidifie lorsqu'elle est appliquée sur les lèvres ou la peau ou les phanères, et qui récupère toute ou partie de sa viscosité initiale au cours du temps après l'application, à température ambiante.

Il est connu de l'homme du métier d'utiliser dans des compositions cosmétiques et/ou dermatologiques contenant une phase grasse liquide, des organogélateurs moléculaires pour structurer la phase grasse.

Cependant, de telles compositions présentent l'inconvénient de présenter un comportement rhéofluidifiant.

Par comportement rhéofluidifiant, on entend, au sens de l'invention, une composition qui conduit à un dépôt qui fluidifie lors de son application, mais reste fluide et ne récupère pas toute ou partie de sa viscosité initiale ou seulement après un temps très long (un mois ou plus).

Par ailleurs, l'utilisation d'organogélateurs moléculaires pour structurer la phase grasse conduit après application à des dépôts fracturants, à savoir des dépôts granuleux ou sous forme de petits morceaux ou écailles.

En outre, une composition cosmétique présentant un comportement rhéofluidifiant peut entraîner l'exsudation de la phase grasse, notamment dans les régions chaudes et humides. Une telle composition peut également favoriser, après dépôt sur la peau ou sur les lèvres, la migration de cette phase dans les rides et ridules de la peau qui entourent notamment les lèvres et les yeux, ce qui est particulièrement gênant pour les rouges à lèvres et les fards à paupières.

En effet, une migration importante de la phase grasse liquide, en particulier lorsqu'elle est chargée de matières colorantes, entraîne avec elle des matières colorantes le cas échéant et conduit à un effet inesthétique autour des lèvres et des yeux, accentuant particulièrement les rides et les ridules.

Par migration, on entend, au sens de l'invention, un débordement de la composition, et en particulier de la couleur, hors du tracé initial du maquillage.

Il subsiste donc le besoin de compositions ne présentant pas les inconvénients ci-dessus, et notamment des compositions conduisant après application à des dépôts

- qui récupèrent leur viscosité initiale dans un temps très court après application, en d'autres termes qui se solidifient rapidement,
- qui ne migrent pas dans les rides et ridules de la peau ou des lèvres,
- qui ne fracturent pas et ne sont pas granuleux, hétérogènes, mais au contraire sont homogènes, avec une texture lisse et continue,
- qui présentent une facilité d'étalement et des propriétés d'écoulement.

En outre, la composition après cisaillement est onctueuse crémeuse.

La demanderesse a découvert de manière surprenante que l'utilisation d'un additif polaire, dans une composition cosmétique comprenant une phase grasse liquide, structurée par au moins un organogélateur moléculaire permettait de modifier la rhéologie de la composition, et en particulier de lui conférer un caractère thixotrope.

Par caractère thixotrope de la composition, on entend, au sens de l'invention, la propriété de la composition de se fluidifier sous cisaillement, et en particulier lors de l'application sur la peau, les lèvres ou les phanères, et de revenir totalement ou en partie à sa viscosité d'origine, au cours du temps et à température ambiante.

Plus particulièrement, la demanderesse a découvert que cet additif polaire ne donne un caractère thixotrope à la composition, que si la phase grasse contient au moins une huile apolaire ou faiblement polaire. L'additif polaire peut être choisi parmi les composés considérés comme de bons donneurs ou accepteurs de liaisons H, comme par exemple les alcools gras, les acides gras, les diols, les esters, les pyrrolidones, les cétones ou les sulfoxides.

L'homme de l'art connaît l'utilisation de ces additifs polaires comme solvant ou agent de couplage dans un but de compatibilisation des compositions.

Ainsi, à titre de document illustratif, on peut citer la publication "Method of Gelling Non-polar Oils with Dibenzylidène Sorbitol (DBS) through the Use of Co-solvents" de Dimitris E. Katsoulis et Janet M. Smith (Research Disclosure, August 1994, Dow Corning Corporation). Ce document décrit une méthode de gélification d'huile de silicone avec le dibenzylidène sorbitol (DBS) et un co-solvant polaire, pour fabriquer des gels à fort taux de silicone. Le co-solvant polaire est utilisé pour compatibiliser l'huile de silicone à gélifier et le DBS et rend donc possible la gélification de l'huile de silicone par le DBS. Les gels de silicone obtenus par cette méthode peuvent être utilisés en tant que véhicules pour des sels antitranspirants, des pigments et des écrans solaires. Ils peuvent également être utilisés sous forme de gels ou sticks cosmétiques, ou sous forme de gels ou sticks antitranspirants ou déodorants, ou encore sous forme de gels ou sticks écrans solaires.

Le brevet américain US-5403580 décrit une méthode de gélification d'huile de silicone avec un gélifiant dérivé de stéroïde et un co-solvant utilisé pour dissoudre le gélifiant dans l'huile de silicone, le co-solvant étant un solvant volatile tel que le chloroforme ($CHCl_3$).

Par ailleurs, le brevet américain US-5403580 décrit une composition pour gel antitranspirant sous forme de stick, comprenant un actif antitranspirant, un premier gélifiant choisi dans le groupe de l'acide hydroxystéarique et de ses dérivés (notamment esters et amides), un deuxième gélifiant choisi dans le groupe du N-acylaminoacide amide et de ses dérivés, et un co-solvant polaire. Le co-solvant polaire permet d'augmenter la compatibilité des gélifiants et de l'huile de silicone, ce qui conduit à une température de mise en oeuvre plus basse et une plus grande proportion d'huile dans le stick. Le co-solvant est choisi parmi les alcools gras, les acides gras, les éthers et les esters dérivés des alcools et acides, et notamment l'octyl dodécanol, l'undécyl pentadécanol, l'éthyl myristate, l'isopropyl palmitate, le finsolv et le diisopropyl sébaçate.

Enfin, le brevet américain US-5958386 décrit une composition antitranspirante comprenant un actif antitranspirant, une huile hydrocarbonée volatile, un gélifiant choisi parmi les acides gras, les esters d'acides gras, les amides d'acides gras et les dérivés du N-acylamino acide, et un agent de couplage pour compatibiliser le gélifiant et l'huile hydrocarbonée et diminuer ainsi la température de mise en oeuvre. Les agents de couplage sont de préférence choisis parmi le diméthiconol, l'octyldodécanol, l'hexyldécanol, l'octydécanol, l'undécylpentadécanol, l'isopropylmyristate, le diisopropyl adipate, l'éthanol et le diéthylphtalate.

Aucun de ces documents ne concerne l'utilisation d'un additif polaire dans une composition structurée comprenant une huile apolaire ou faiblement polaire et un organogélateur moléculaire, pour modifier la rhéologie de cette composition en lui conférant un caractère thixotrope.

Toutefois, la demande internationale WO-00015180-A1 décrit une composition structurée (épaissie ou gélifiée) comprenant une huile ou une cire non polaire, un composé pour modifier la rhéologie (gélifiant), de préférence un polysaccharide, et un composé à liaisons H pour compatibiliser l'huile et le gélifiant. Le gélifiant n'est pas un organogélateur moléculaire. De plus, ce document enseigne que la viscosité de la composition varie en fonction de la proportion d'agent de couplage dans la composition, mais rien n'est dit concernant une modification profonde de la rhéologie en fonction de la proportion d'agent de couplage, et en particulier la modification du comportement rhéologique de la composition structurée vers un comportement thixotrope lorsqu'elle contient un additif polaire.

L'invention a donc pour objet l'utilisation d'au moins un additif polaire ayant un paramètre de polarité $\delta_a$ égal ou supérieur à 7,0 $(J/cm^3)^{1/2}$, dans une composition cosmétique comprenant une phase grasse liquide contenant au

moins une huile apolaire ou faiblement polaire ayant un paramètre de polarité inférieur $\delta_a$ à 7,0 $(J/cm^3)^{1/2}$, structurée par au moins un organogélateur moléculaire, pour conférer un caractère thixotrope à la composition.

En outre, l'utilisation d'au moins un additif polaire tel que défini ci-dessus, dans une composition cosmétique permet également :

- de limiter, voire supprimer la migration de la phase grasse dans les rides et ridules de la peau ou des lèvres ;
- de limiter la formation de stries sur les paupières notamment lorsque la composition est un fard à paupières ;
- de limiter, voire supprimer le comportement fracturant caractéristique des compositions structurées par un organogélateur moléculaire, et/ou favoriser l'étalement de la composition sur la peau et/ou former un dépot sur les matières kératiniques lisse, homogène, non granuleux, et continu.
- de favoriser l'étalement de la composition sur la peau ou les lèvres.

La structuration de la phase grasse liquide est modulable selon la nature de l'organogélateur moléculaire utilisée, et la rhéologie de la composition est parfaitement ajustable par adaptation de la quantité d'additif utilisée.

<u>Additif polaire</u>

L'additif polaire selon l'invention est un bon donneur ou un bon accepteur de liaisons hydrogène, choisi parmi les alcools gras, les acides gras, les diols les esters, les éthers, les pyrrolidones, les cétones, les sulfoxydes, et leurs mélanges.

De préférence, l'additif polaire selon l'invention est choisi parmi l'octyldodécanol, l'hexyldécanol, l'octyldécanol, l'undécylpentadécanol, l'hexylène glycol le 1,2-hexanediol, le propylène glycol, la glycérine, l'alcool oléique, l'alcool phényléthylique, l'huile de ricin, l'éthyl-myristate, l'isopropylpalmitate, le Finsolv, le diisopropyl-sébaçate, le diisopropyladipate, le propylène glycol dicaprate/dicaprylate et leurs mélanges.

Avantageusement, l'additif polaire est présent dans la composition à raison de 1 à 40% en poids, de préférence de 5 à 30% en poids, et mieux de 8 à 20% en poids par rapport au poids total de la composition.

De façon générale, la quantité d'additif polaire croît avec la quantité d'organogélateur et la nature (apolaire ou faiblement polaire) de la phase grasse liquide ; la quantité d'additif doit être suffisante pour conférer un caractère thixotrope à la composition et ne pas solubiliser totalement l'organogélateur à la température ambiante.

<u>Organogélateur</u>

Selon l'invention, la composition comprend un ou plusieurs organogélateurs moléculaires.

Les molécules d'organogélateurs sont capables d'établir, entre elles, des interactions physiques conduisant à une auto-agrégation des molécules avec formation d'un réseau supra-moléculaire 3D qui est responsable de la gélification de la phase grasse liquide. Le réseau peut résulter de la formation d'un réseau de fibrilles (dues aux empilements ou agrégations de molécules d'organogélateur), immobilisant les molécules de la phase grasse liquide. Selon la nature de l'oganogélateur, les fibrilles interconnectées ont des dimensions variables pouvant aller jusqu'au µm, voire plusieurs µm. Ces fibrilles peuvent parfois s'associer pour former des rubans ou colonnes.

L'aptitude à former ce réseau de fibrilles, et donc la gélification, dépend de la nature (ou classe chimique) de l'organogélateur, de la nature des substituants portés par ses molécules pour une classe chimique donnée et de la nature de la phase grasse liquide.

Les interactions physiques sont diverses mais excluent la co-cristallisation. Ces interactions physiques sont en particulier des interactions du type interactions hydrogènes auto-complémentaires, interactions $\pi$ entre cycles insaturés, interactions dipolaires, liaisons de coordination avec des dérivés organométalliques et leurs associations. L'établissement de ces interactions est souvent favorisé par l'architecture de la molécule : cycles, insaturations, présence de carbones asymétriques. En général, chaque molécule d'un organogélateur peut établir plusieurs types d'interactions physiques avec une molécule voisine. Aussi, avantageusement, les molécules des organogélateurs selon l'invention comportent au moins un groupement capable d'établir des liaisons hydrogènes et mieux au moins deux groupements capables de faire des liaisons hydrogènes, au moins un cycle aromatique et mieux aux moins deux cycles aromatiques, au moins une ou plusieurs liaisons à insaturation éthylénique et/ou au moins un ou plusieurs carbones asymétriques. De préférence, les groupements capables de faire des liaisons hydrogènes sont choisis parmi les groupements hydroxyle, carbonyle, amine, acide carboxylique, amide, urée, benzyle et leurs associations.

Le ou les organogélateurs selon l'invention sont solubles dans la phase grasse liquide après chauffage jusqu'à obtention d'une phase liquide homogène transparente. Ils peuvent être solides ou liquides à température ambiante et pression atmosphérique.

Le ou les organogélateurs moléculaires utilisables dans l'invention sont notamment ceux décrits dans le document "Specialist Surfactants", édité par D. Robb de 1997, p.209-263, chapitre 8 de P. Terech, les demandes de

brevets françaises non encore publiées 9909178, 0009317. Ce sont plus spécialement des acides gras carboxyliques hydroxylés à chaîne carbonée aliphatique linéaire ou ramifiée comportant notamment au moins 8 atomes de carbone et mieux au moins 12 atomes de carbone comme l'acide 12-hydroxystéarique ou l'acide 12-hydroxyoléique et leurs sels de métaux alcalins (Li, Na ou K notamment) ou alcalino-terreux (Mg par exemple) ; des amides d'acides carboxyliques en particulier tri-carboxyliques comme les cyclohexane tricarboxamides résultant de la réaction de l'acide cyclohexane tricarboxylique et d'une lauryle amine (voir la demande de brevet français n°9909178), ces amides répondant notamment à la formule (I) ci-après ; des dérivés uréides comme les dérivés du 1,2-bis(uréido)benzène et du *trans*-1,2-bis (uréido)cyclohexane, et en particulier ceux décrits dans l'article de R. M. Kellog, B. L. Feringa et al. "Cyclic Bis-Urea Compounds as gelators for Organic Solvents" (*Chem. Eur. J.* 1999. 5. N°3) ; des amides ou esters d'amino acides comme les esters de l'analine et les amides de la valine, et en particulier ceux décrits dans le livre "Specialist Surfactants" ; les N-Acyl Aminoacides et leurs dérivés, et notamment les amides de N-acylamino acides comme les diamides résultant de l'action d'un N-acylamino acide avec des amines comportant de 1 à 22 atomes de carbone, comme par exemple celles décrites dans la demande internationale WO-93/23008 et notamment les amides de l'acide N-acylglutamique où le groupe acyle représente une chaîne alkyle en $C_8$ à $C_{22}$ ; les diamides ayant de 1 à 22 atomes de carbone, et mieux de 6 à 18 atomes, les chaînes hydrocarbonées étant éventuellement substituées par des groupes ester, urée, fluorés (voir la demande de brevet français n°0009317), ces diamides sont en particulier ceux de formule (II) ci-après ; les aminés ou amides de stéroïde et notamment d'acide déoxycholique, cholique, apocholique, lithocholique et leurs sels comme le D-17,17-dipropyl-17a-aza-5α-homoandrostan-3ol ou le D-17,17-dipropyl-17*a*-aza-5α-homoandrostan-3β-ol 17*a*-oxy ; les composés à plusieurs cycles aromatiques et notamment les dérivés anthryle comportant au moins 2 chaînes alkyle ayant de 8 à 30 atomes de carbone comme le 2,3-bis-n-décyloxyanthracène, la 2,3-bis-n- décyloxyanthraquinone ou comportant un groupement stéroïde comme le cholestéryl 4-(2-anthryloxy)butanoate ou le cholestéryl anthraquinone -2- carboxylate et leurs dérivés ; les azobenzène stéroïdes tels que ceux décrits dans le livre "Specialist Surfactants" ; les composés organométaliques comme le β-dicétonate de cuivre mononucléaire (le complexe de cuivre octasubstitué de bis(3,4 nonyloxy benzoyl) méthanes), les tétracarboxylates de cuivre binucléaire ou les complexes du Zn (II) de (para-carboxyphényl) porphyrine trisubstitué ; les tensioactifs sous forme de sel comportant au moins deux chaînes alkyles linéaires ou ramifiées et en particulier les alkylphosphates de métaux alcalins ou d'aluminium comportant deux chaînes alkyles ayant de 8 à 30 atomes de carbone comme le sel d'aluminium de dihexadécyl phosphate ($C_{16}$) ou l'acide phosphorique de di(2-éthyl hexyle) et ses sels de métaux alcalins (Na), l'acide sulfosuccinique de di(2-éthyl hexyle) et ses sels de métaux alcalins (Na) ; les benzylidènes sorbitols ou alditols et dérivés comme le 1,3 : 2,4-di-*o*-benzylidène-D-sorbitol, et leurs mélanges.

De préférence, on utilise les amides d'amino acides et plus spécialement les N-acylamino acides et leurs dérivés, les cyclohexane tricarboxamides décrits par la formule (I), les diamides décrits par la formule (II) et leurs mélanges.

*Organogélateur de formule (I)*

Selon l'invention, l'organogélateur peut être un composé de formule (I) suivante :

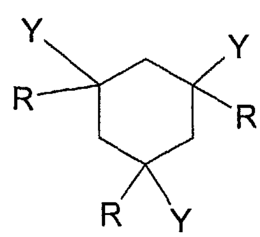

dans laquelle :

- R représente, indépendamment les uns des autres, un atome d'hydrogène ou une chaîne hydrocarbonée saturée, linéaire ou ramifiée, ayant 1 à 6 atomes de carbone, notamment 1 à 4 atomes de carbone;
- Y représente un groupement choisi parmi les groupements suivants : -CO-S-R'; -CO-NHR'; -NH-COR' et -S-COR'; dans lesquels R' représente, indépendamment les uns des autres :
    - un atome d'hydrogène,
    - un groupement aryle,

- un groupement aralkyle, c'est-à-dire aryle substitué par une chaîne hydrocarbonée saturée, linéaire ou ramifiée, comprenant 1 à 22 atomes de carbone, notamment 10-18 atomes de carbone; ou
- une chaîne hydrocarbonée saturée, linéaire, ramifiée ou cyclique, comprenant 1 à 22 atomes de carbone, notamment 10-18 atomes de carbone, éventuellement substituée par un ou plusieurs groupements choisis parmi les groupements aryle, ester, amide, uréthanne; et/ou comprenant éventuellement un ou plusieurs hétéroatomes choisi parmi O, S et N; et/ou éventuellement substituée par un ou plusieurs atomes de fluor et/ou radicaux hydroxy.

De préférence, R représente un atome d'hydrogène.
De préférence, Y représente un groupement -CO-NHR' ou -NH-COR'.
De préférence, R' représente un groupement aryle; un groupement aralkyle dans lequel la chaîne alkyle, linéaire ou ramifiée, comprend 12-16 atomes de carbone; ou une chaîne alkyle linéaire ou ramifiée en $C_{12}$-$C_{18}$.
Encore plus préférentiellement, Y représente un groupement -CO-NHR' dans lequel R' représente un groupement aryle substitué par une chaîne alkyle linéaire ou ramifiée, en $C_{12}$-$C_{16}$ ; ou R' représente une chaîne alkyle linéaire ou ramifiée, en $C_{12}$-$C_{18}$, non substituée.
Les trois substituants représentés par Y peuvent être, dans les composés de formule (I), en conformation cis-cis, cis-trans ou trans-trans, les uns par rapport aux autres. Notamment, au moins un de ces substituants peut être placé en position équatoriale sur le cycle cyclohexane. De préférence, tous les substituants Y sont placés en position équatoriale.
Parmi les composés de formule (I) susceptibles d'être employés comme organogélateur, seul ou en mélange, dans la composition de l'invention, on peut citer :

- le cis-1,3,5-tris(dodecylaminocarbonyl)cyclohexane,
- le cis-1,3,5-tris(octadecylaminocarbonyl)cyclohexane,
- le cis-1,3,5-tris[N-(3,7-diméthyloctyl)-aminocarbonyl]cyclohexane,
- le trans-1,3,5-triméthyl-1,3,5-tris(dodecylaminocarbonyl)-cyclohexane, et
- le trans-1,3,5-triméthyl-1,3,5-tris(octadecylaminocarbonyl)-cyclohexane.

Les composés de formule (I) sont bien connus de l'homme de l'art et peuvent être préparés selon les procédés usuels.

*Organogélateur de formule (II)*

Selon l'invention, l'organogélateur peut être un composé de formule (II) suivante :

R-CO-NH-A-NH-CO-R'

dans laquelle :

- R et R', identique ou différents, représentent un atome d'hydrogène ou une chaîne hydrocarbonée saturée ou insaturée, linéaire, ramifiée ou cyclique, comprenant 1 à 22 atomes de carbone, notamment 6-18 atomes de carbone, éventuellement substituée par un ou plusieurs groupements choisis parmi les groupements aryle ($-C_6H_5$), ester (-COOR" avec R" ayant 2 à 12 atomes de carbone), amide (-CONHR" avec R" ayant 2 à 12 atomes de carbone), uréthanne (-OCONHR" avec R" ayant 2 à 12 atomes de carbone), urée (-NHCONHR" avec R" ayant 2 à 12 atomes de carbone) ; et/ou éventuellement comprenant 1 à 3 hétéroatomes choisi parmi O, S et N ; et/ou éventuellement substituée par 1 à 4 atomes d'halogènes, notamment de fluor et/ou par 1 à 3 radicaux hydroxy, à la condition que R et/ou R' soit différent de l'hydrogène, et
- A représente une chaîne hydrocarbonée, saturée ou insaturée, linéaire, cyclique ou ramifiée, ayant 1 à 18 atomes de carbone, notamment 2 à 12 atomes de carbone, éventuellement substitué par un ou plusieurs groupements choisis parmi les groupements aryle ($-C_6H_5$), ester (-COOR" avec R" ayant 2 à 12 atomes de carbone), amide (-CONHR" avec R" ayant 2 à 12 atomes de carbone), uréthanne (-OCONHR" avec R" ayant 2 à 12 atomes de carbone), urée (-NHCONHR" avec R" ayant 2 à 12 atomes de carbone) ; et/ou éventuellement comprenant 1 à 3 hétéroatomes choisi parmi O, S et N ; et/ou éventuellement substituée par 1 à 4 atomes d'halogènes, notamment de fluor et/ou par 1 à 3 radicaux hydroxy.

Par chaîne hydrocarbonée insaturée selon la formule (II), on entend une chaîne qui comprend au moins une double liaison c=c ou au moins une triple liaison c≡c, ladite chaîne pouvant bien entendu en outre être éventuellement

substituée par un ou plusieurs groupements choisis parmi les groupements aryle, ester, amide, uréthanne, urée ; et/ou comprendre éventuellement un ou plusieurs hétéroatomes choisi parmi O, S et N ; et/ou éventuellement être substituée par un ou plusieurs atomes de fluor et/ou radicaux hydroxy.

Par chaîne hydrocarbonée selon la formule (II) comprenant un atome d'oxygène, de soufre ou d'azote, on entend notamment une chaîne hydrocarbonée comprenant un groupement carbonyle (-C=O) aminé (-NH2 ou -NH-), thiol (-SH), thioéther ou éther.

De préférence, les composés répondent à la formule (II) dans laquelle :

***1/***

- A représente un cycle hydrocarboné, saturé ou insaturé mais non aromatique, éventuellement ramifié, ayant 4 à 12 atomes de carbone, notamment 5 à 7 atomes de carbone, éventuellement substitué par les substituants ci-dessus cités et/ou éventuellement comprenant 1 ou plusieurs hétéroatomes et/ou éventuellement substitué par 1 ou plusieurs halogènes et/ou radicaux hydroxy ;
- R et R', identiques ou différents, représentent un atome d'hydrogène ou une chaîne hydrocarbonée saturée ou insaturée, linéaire, ramifiée ou cyclique, de préférence saturée linéaire, comprenant 10 à 16 atomes de carbone, notamment 12 à 14 atomes de carbone ; ou

***2/***

- A représente une chaîne hydrocarbonée saturée, linéaire ou ramifiée, ayant 2 à 18 atomes de carbone, notamment 3 à 12 atomes de carbone, éventuellement substituée par les substituants ci-dessus cités, et/ou éventuellement comprenant 1 ou plusieurs hétéroatomes et/ou éventuellement substituée par 1 ou plusieurs halogènes et/ou radicaux hydroxy ;
- R et R', identiques ou différents, représentent un atome d'hydrogène ou une chaîne hydrocarbonée saturée ou insaturée, linéaire, ramifiée ou cyclique, de préférence saturée linéaire, comprenant 10 à 20 atomes de carbone, notamment 11 à 18 atomes de carbone, de préférence 11 à 13 atomes de carbone, et mieux 11 atomes de carbone, à la condition que R et R' soient différents de l'hydrogène

ou encore

**3/**

- A représente un cycle aryle ou aralkyle ayant 4 à 12 atomes de carbone, notamment 5 à 8 atomes de carbone, éventuellement substitué par les substituants ci-dessus cités et/ou éventuellement comprenant 1 ou plusieurs hétéroatomes et/ou éventuellement substituée par 1 ou plusieurs halogènes et/ou radicaux hydroxy ;
- R et R', identiques ou différents, représentent un atome d'hydrogène ou une chaîne hydrocarbonée saturée ou insaturée, linéaire, ramifiée ou cyclique, de préférence saturée linéaire, comprenant 6 à 18 atomes de carbone, notamment 10 à 16 atomes de carbone, de préférence comprenant 11 à 13 atomes de carbone, et mieux 11 atomes de carbone, à la condition que R et R' soient différents de l'hydrogène.

Notamment, le radical A peut représenter un radical divalent de type cyclohexylène (en particulier 1,2-cyclohexylène, 1,3-cyclohexylène, 1,4-cyclohexylène ; de préférence 1,2-cyclohexylène), éthylène, propylène, isopropylène, butylène, isobutylène, pentylène, hexylène, dodécylène, dodécanylène, benzylène, phénylène, méthyl-phénylène, bis-phénylène ou napththalène, de préférence A peut être un radical divalent de type cyclohexylène, éthylène, propylène, isopropylène, dodécylène, méthyl-phénylène.

Les radicaux R et R' peuvent, indépendamment l'un de l'autre, être choisis parmi les radicaux pentyle, hexyle, décyle, undécyle, dodécyle, pentadécyle, hexadécyle, heptadécyle, octadécyle, 3-dodécyloxyproprionyle, 3-octadécyloxyproprionyle, 3-dodécyloxypentyle, 3-octadécyloxypentyle, 11-hydroxyheptadécyle. De préférence, R et R' peuvent indépendamment l'un de l'autre être choisis parmi les radicaux décyle, undécyle, dodécyle.

Avantageusement, R et R' sont identiques.

Lorsque le radical A est cyclique, les radicaux R-CO-NH- et R'-CO-NH- peuvent être en position ortho, méta ou para. Par ailleurs, ils peuvent être en position cis ou trans l'une par rapport à l'autre.

Préférentiellement, les composés de formule (II) sont choisis parmi les composés répondant à l'une des formules suivantes :

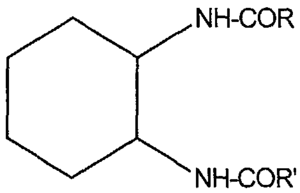

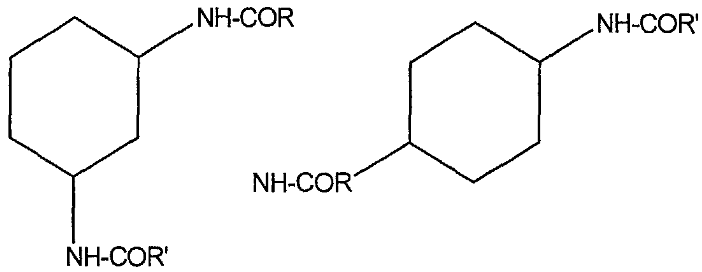

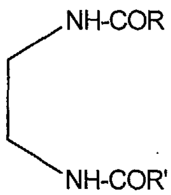

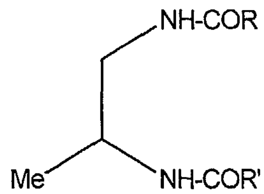

dans laquelle R et R' ont les mêmes significations que ci-dessus.

Parmi les composés susceptibles d'être employés comme organogélateur dans la composition de l'invention, on peut citer :

- le N,N'-bis (dodécanoyl)-1,2-diaminocyclohexane, en particulier sous forme trans (composé de formule (I) avec R=R'=n-$C_{11}H_{23}$ et A = radical divalent 1,2- cyclohexylène, nommé également acide (2-dodécanoylamino-cyclohexyl)-amide dodécanoïque). Ce composé est notamment décrit dans Hanabusa, K. Angew. Chem., 108, 1997, 17, pages, 2086-2088.
- le N,N'-bis (dodécanoyl)-1,3-diaminocyclohexane, en particulier sous forme trans(composé de formule (I) avec R=R'=n-$C_{11}H_{23}$ et A = radical divalent 1,3- cyclohexylène, nommé également acide (3-dodécanoylamino-cyclohexyl)-amide dodécanoïque),
- le N,N'-bis (dodécanoyl)-1,4-diaminocyclohexane, en particulier sous forme trans(composé de formule (I) avec R=R'=n-$C_{11}H_{23}$ et A = radical divalent 1,4- cyclohexylène, nommé également acide (4-dodécanoylamino-cyclohexyl)-amide dodécanoïque),
- le N,N'-bis (dodécanoyl)-1,2-éthylènediamine (composé de formule (I) avec R=R'=n-$C_{11}H_{23}$ et A = radical divalent 1,2-éthylène, nommé également acide (2-dodécanoylamino-éthyl)-amide dodécanoïque),
- le N,N'-bis (dodécanoyl)-1-méthyl-1,2-éthylènediamine (composé de formule (I) avec R=R'=n-$C_{11}H_{23}$ et A = radical divalent 1-méthyl, -1,2- éthylène, nommé également acide (2-dodécanoylamino-2-méthyl-éthyl)-amide dodécanoïque),
- le N,N'-bis (dodécanoyl)-1,3-diaminopropane (composé de formule (I) avec R=R'=n-$C_{11}H_{23}$ et A = radical divalent 1,3-propylène, nommé également acide (2-dodécanoylaminopropyl)-amide dodécanoïque),
- le N,N'-bis (dodécanoyl)-1,12-diaminododécane (composé de formule (I) avec R=R'=n-$C_{11}H_{23}$ et A = radical divalent 1,12-dodécylène, nommé également acide (2-dodécanoylaminododécyl)-amide dodécanoïque),
- le N,N'-bis (dodécanoyl)-3,4-diaminotoluène (composé de formule (I) avec R=R'=n-$C_{11}H_{23}$ et A = radical divalent 1-méthyl-3,4-phénylène, nommé également acide (2-dodécanoylamino-4-méthyl-phenyl)-amide dodécanoïque),
- leurs mélanges.

Les composés de formule (II) peuvent être préparés selon des procédés bien connus de l'homme du métier et être employés, seuls ou en mélange, dans la composition de l'invention.

*Préparation-type des composés de formule (II)*

On dissout la diamine et deux équivalents de triéthylamine dans 50 ml de tétrahydrofuranne. On ajoute deux équivalents de chlorure d'acyle en solution dans le THF, on chauffe le mélange réactionnel au reflux du tétrahydrofuranne en suivant la disparition du chlorure d'acyle par spectroscopie infrarouge (le plus classiquement, deux heures). On filtre la solution du précipitat, on concentre la phase organique et on réalise une extraction liquide/liquide du composé solide obtenu. La phase organique est ensuite séchée puis concentrée et le produit solide obtenu est recristallisé.

*Les N-acylamino acides et leurs dérivés*

Des exemples de N-Acyl-amino acides et de leurs dérivés sont décrits dans le brevet américain US 3,969,087.
Ces N-Acyl-amino acides et leurs dérivés sont des composés de formule générale (III) :

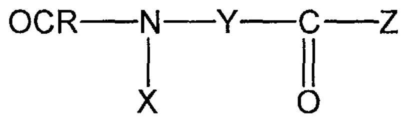

(III)

dans laquelle :

- RCO représente un groupement acyle, comprenant de 2 à 30 atomes de carbone, ou un groupement aralkyle acyle,
- Z représente un groupement hydroxy, NH2, $-OR_1$, $-NRH_2$,

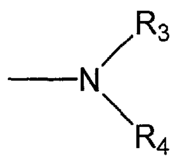

et

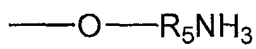

où $R_1$ à $R_5$ représentent des chaînes hydrocarbonées, indépendantes les unes des autres et comprenant de 1 à 30 atomes de carbone, ou des groupements aralkyles,
- X représente un atome d'hydrogène ou un groupement méthyle, et
- Y est une chaîne hydrocarbonée ou un groupement benzyle, phényle, ou
$-CH_2OH$,

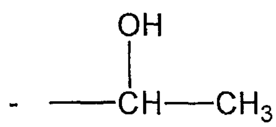

,

ou
$-CH_2SH$, $-CH_2CH_2S-CH_3$, ou
$-(CH_2)_p-COZ$ où p est un entier positif choisi parmi les nombres 1 et 2, et Z est défini tel que précédemment, ou
$-(CH2)_n-$ où n est un entier positif choisi entre 1 et 6 à la condition que Z représente $OR_1$, Y représente

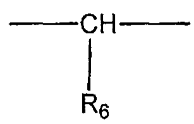

,

et l'un au moins des groupements $R_1$ à $R_5$ est une chaîne hydrocarbonée.

De manière préférée, RCO est un groupement acyle comprenant de 12 à 30 atomes de carbone, X est un atome d'hydrogéné et Y est

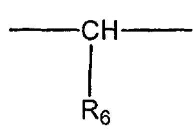

où $R_6$ représente $-(CH_2)_p-COZ$ où Z est $NHR_2$.

Les organogélateurs particulièrement préférés sont les amides des N-Acylaminoacides de formule générale (IV):

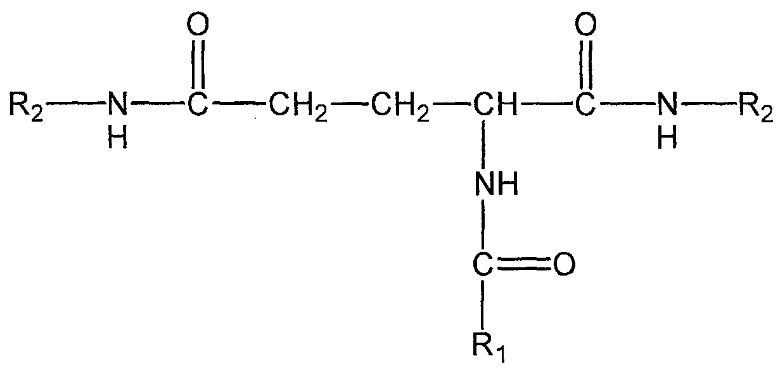

(IV)

où $R_1$ est une chaîne hydrocarbonée comprenant de 12 à 22 atomes de carbone et $R_2$ est une chaîne hydrocarbonée comprenant de 2 à 22 atomes de carbone.

Les aminoacides peuvent être des isomères D ou L, mais les isomères L sont préférés.

A titre d'exemple de composés ayant la formule générale (IV), on peut citer le dibutylamide de l'acide n-lauroyl-L-glutamique, le diheptylamide de l'acide n-stéaroyl-L-glutamique, le diéthylamide de l'acide N-Lauroyl-L-glutamique, le dioctylamide de l'acide N-Lauroyl-L-glutamique, le didécylamide de l'acide N-Lauroyl-L-glutamique, l'acide N-Lauroyl-L-glutamique, le ditétradécylamide, etc.

L'organogélateur de formule (IV) préféré est le dibutylamide de l'acide N-Lauroyl-L-glutamique, commercialisé par la société Ajinomoto sous la dénomination GP-1 ou celui commercialisé par la société CLARIANT sous le nom de LGB.

Les esters des N-Acylamino acides de formule générale (IV) sont également des organogélateurs préférés selon l'invention.

A titre d'exemples de tels esters, on peut citer les esters de N-lauroyl-□-alanine, de N-lauroyl-valine, de l'acide N-lauroyl-L-glutamique, de N□N□-dicaproyl-ornithine, de N□N□-dicaproyl-ornithine-octyle, décyle, lauryle et stéaryle, et de N□N□-dicaproyllysine-octyle, décyle, et lauryle.

La composition contient avantageusement de 0,1 à 60 % en poids d'organogélateur, de préférence de 1 à 30 % en poids, et mieux de 2 à 20 % en poids par rapport au poids total de la composition.

Phase grasse

La phase grasse liquide selon l'invention peut contenir une ou plusieurs huiles liquides apolaires ou faiblement polaires.

Les huiles apolaires selon l'invention sont en particulier les huiles siliconées telles que les polydiméthylsiloxanes (PDMS) volatils ou non, linéaires ou cycliques, liquides à température ambiante ; les polydiméthylsiloxanes comportant des groupements alkyle ou alcoxy, pendant et/ou en bout de chaîne siliconée, groupements ayant chacun de 2 à 24 atomes de carbone ; les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ; les hydrocarbures linéaires ou ramifiés d'origine synthétique ou minérale volatile ou non comme les huiles de paraffine, volatiles (isoparaffines ou isododécane) ou non volatiles, et ses dérivés, la vaseline, la lanoline liquide, les polydécènes, le polyisobutène hydrogéné tel que le Parléam (de chez NIPPON Gel Fats), le squalane ; les huiles fluorées notamment perfluorées ; les silicones fluorées; et leurs mélanges.

De préférence, les huiles apolaires selon l'invention sont des huiles ou des mélanges d'huiles du type hydrocarboné d'origine minérale ou synthétique, choisies en particulier parmi les hydrocarbures notamment les alcanes comme l'huile de Parléam, les isoparaffines comme l'isododécane et le squalane et leurs mélanges.

Par huiles du type hydrocarboné, on entend, au sens de l'invention, des huiles ne comportant que des atomes d'hydrogène et de carbone.

Les huiles faiblement polaires selon l'invention, ayant un paramètre de polarité $\delta_a$ inférieur à 7,0 $(J/cm^3)^{1/2}$, sont choisies parmi les esters d'acide et/ou d'alcool gras en $C_8$-$C_{70}$ tels que le palmitate d'éthyle hexyle, le trimellitate de tridécyle, le myristate d'isopropyle, le myristate de myristyle, le myristate d'octyldodécyle, le stéarate de butyle, le stéarate d'isopropyle, le stéarate d'éthyl-hexyle, le stéarate d'octyldodécyle, l'heptanoate de stéaryle, le caprylate de

stéaryle, l'adipate de diisopropyle, le néopentanoate d'isostéaryle, l'isostéarate d'isoprypyle, l'isononanoate d'isooctadécyle, l'isononanoate d'isononyle.

La phase grasse contient :

- soit au moins une huile apolaire présente dans la composition à raison de 0,2 à 98,9% en poids, de préférence à raison de 1 à 80% en poids, et mieux de 5 à 60% en poids par rapport au poids de la composition grasse,
- soit au moins une huile peu polaire ayant un paramètre de polarité $\delta_a$ inférieur à 7,0 $(J/cm^3)^{1/2}$, présente dans la composition à raison de 0,2 à 98,9% en poids, de préférence à raison de 1 à 80% en poids, et mieux de 5 à 60% en poids par rapport au poids de la phase grasse.

La phase grasse liquide totale représente, en pratique, de 0,2 à 98,9% en poids, de préférence de 10 à 80% en poids, et mieux de 5 à 60% en poids du poids total de la composition. Elle peut ne contenir que des huiles apolaires, ou des huiles peu polaires, ou un mélange de ces huiles.

La composition selon l'invention peut, en outre, comprendre une ou plusieurs huiles polaires, à savoir des huiles ayant un paramètre de polarité $\delta_a$ égal ou supérieur à 7,0 $(J/cm^3)^{1/2}$, constituant en particulier l'additif polaire, choisies parmi l'octyldodécanol, l'hexyldécanol, l'octyldécanol, l'undécylpentadécanol, l'hexylène glycol, le 1,2-hexanediol le propylène glycol, la glycérine, l'alcool oléique, l'alcool phényléthylique, l'huile de ricin, l'éthyl-myristate, l'isopropyl-palmitate, le Finsolv, le diisopropyl-sébaçate, le diisopropyl-adipate, le propylène glycol dicaprate/dicaprylate et leurs mélanges.

L'invention a encore pour objet une composition cosmétique comprenant au moins une phase grasse liquide contenant au moins une huile hydrocarbonée apolaire ou faiblement polaire, ayant un paramètre de polarité $\delta_a$ inférieur à 7,0 $(J/cm^3)^{1/2}$, structurée par au moins un organogélateur moléculaire de la phase grasse liquide à l'exclusion de tout gélifiant moléculaire de masse moléculaire moyenne en poids allant de 1000 à 100.000 et comportant un squelette polymérique ayant des motifs urée, uréthane, thiourée, thiouréthane et amide, et au moins un additif polaire ayant un paramètre de polarité $\delta_a$ égal ou supérieur à 7,0 $(J/cm^3)^{1/2}$, la phase grasse, l'organogélateur et l'additif formant un milieu physiologiquement acceptable.

L'additif polaire de la composition selon l'invention est tel que défini ci-dessus.

L'organogélateur moléculaire de la composition selon l'invention est tel que défini ci-dessus et de préférence choisi parmi les N-acylamino acides, les amides de N-acylamino acides, les cyclohexane tricarboxiamides, les cyclohexane diamides, et leurs mélanges.

Les huiles hydrocarbonées apolaires de la composition selon l'invention sont avantageusement choisies parmi le polyisobutène hydrogéné tel que le Parléam (commercialisé par Nippon Gel Fats), le squalane, les hydrocarbures aliphatiques notamment en $C_6$-$C_{40}$ et leurs mélanges.

Comme huiles apolaires préférées selon l'invention, on peut citer les silicones comme les phényltriméticones et la cyclopentadiméthylsiloxane.

Les huiles hydrocarbonées faiblement polaires préférés de la composition selon l'invention sont des huiles ayant un paramètre de polarité $\delta_a$ inférieur à 7,0 $(J/cm^3)^{1/2}$, choisies parmi le palmitate d'éthyle hexyle, le trimellitate de tridécyle, l'isononanoate d'isononyle, le myristate d'isopropyle et leurs mélanges. Les constituants de la formule sont ceux décrits précédemment.

La composition, selon l'invention, présente une texture et une rhéologie particulière.

En effet, l'ajout d'additif polaire ne modifie pas la capacité à gélifier de l'organogélateur dans les huiles polaires ou peu polaires conformes à l'invention.

Toutefois, l'ajout progressif de l'additif polaire induit un renforcement de la gélification qui se traduit par une augmentation de la consistance (dureté) des compositions jusqu'à une concentration maximale $C_{max}$ en additif polaire. Au-delà de $C_{max}$, la consistance et le caractère solide des compositions diminuent. En effet, la compatibilité de l'organogélateur avec la phase grasse est fortement augmenté, une dissolution partielle de l'organogélateur se produit et de ce fait, la composition est moins consistante et moins solide.

Par "solide", on entend, au sens de l'invention, une composition qui ne s'écoule pas sous son propre poids à température ambiante. Par "augmentation de la compatibilité", on entend une solubilisation de l'organogélateur dans la phase grasse liquide à plus basse température.

De plus, grâce à l'additif polaire, les propriétés sous cisaillement de la composition de l'invention sont améliorées. En particulier, on observe une meilleure fluidification de la composition (diminution lente et régulière de la viscosité apparente des compositions sous l'effet du cisaillement) et notamment un meilleur étalement sur les matières kératiniques. Le phénomène de fracturation (effet petit bout) du dépôt de la composition, obtenu en l'absence de l'additif polaire, est diminué ; les textures obtenues après un fort cisaillement sont plus onctueuses et plus homogènes. En outre, les qualités de délitement des compositions sont fortement améliorées avec l'ajout d'une quantité efficace d'additif polaire.

Par ailleurs, les aptitudes à la restructuration des compositions après leur étalement varient selon la nature de l'huile et la quantité d'additif polaire ajoutée. Les propriétés de reprise de viscosité et de consistance du dépôt de la composition sont considérablement améliorées par l'ajout d'additif polaire dans l'association {organogélateur + huile}. Au fur et à mesure que la concentration en additif polaire augmente les propriétés de reprise de viscosité et de consistance sont améliorées, jusqu'à $C_{max}$ où la composition contenant de l'additif polaire possède un niveau de consistance après cisaillement 10 fois plus important que celui de la composition ne contenant pas l'additif polaire. L'intensité de ce phénomène (propriétés de reprise et restructuration) est légèrement moins importante dans le Parléam que dans le palmitate d'éthyle2-hexyle et encore moins dans le trimellitate de tridecyle, où les cinétiques de reprise sont plus lentes, bien que satisfaisantes.

La composition de l'invention peut comprendre, en outre, tout additif complémentaire usuellement utilisé dans le domaine concerné et en particulier cosmétique ou dermatologique, choisi notamment parmi, les antioxydants, les huiles essentielles, les conservateurs (BHT), les parfums, les charges (Polytrap® de chez Dow Corning), les neutralisants, les gommes, les polymères liposolubles ou dispersibles dans le milieu autres que les polyurées, polyuréthanes, polythiourées et polythiouréthanes, les actifs cosmétiques ou dermatologiques comme par exemple les émollients, les hydratants, les vitamines, les acides gras essentiels, les dispersants comme l'acide poly(12-hydroxystéarique) et leurs mélanges.

Chaque additif peut être présent dans la composition à raison de 0 à 20% (notamment de 0,01 à 20 %) du poids total de la composition, et mieux de 0,01 à 10% (si présents).

La composition selon l'invention peut contenir, en outre, au moins un composé gras pâteux à température ambiante. Par " corps gras pâteux " au sens de l'invention, on entend des corps gras ayant un point de fusion allant de 20 à 55 °C, de préférence 25 à 45°C, et/ou une viscosité à 40 °C allant de 0,1 à 40 Pa.s (1 à 400 poises), de préférence 0,5 à 25 Pa.s, mesurée au Contraves TV ou Rhéomat 80, équipé d'un mobile tournant à 200 tr/min. L'homme du métier peut choisir le mobile permettant de mesurer la viscosité, parmi les mobiles MS-r3 et MS-r4, sur la base de ses connaissances générales, de manière à pouvoir réaliser la mesure du composé pâteux testé.

Parmi les composés pâteux susceptibles d'être utilisés dans la composition selon l'invention, on peut citer les lanolines et les dérivés de lanoline comme les lanolines acétylées ou les lanolines oxypropylènées ou le lanolate d'isopropyle, ayant une viscosité de 18 à 21 Pa.s, de préférence 19 à 20,5 Pa.s, et/ou un point de fusion de 30 à 55°C et leurs mélanges. On peut également utiliser des esters d'acides ou d'alcools gras, notamment ceux ayant 20 à 65 atomes de carbone (point de fusion de l'ordre de 20 à 35°C et/ou viscosité à 40 °C allant de 0,1 à 40 Pa.s) comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées (huile de ricin hydrogénée), les polyesters visqueux comme l'acide poly(12-hydroxystéarique) ; les polydiméthylsiloxanes (PDMS) ayant des chaînes pendantes du type alkyle ou alcoxy ayant de 8 à 24 atomes de carbone, et un point de fusion de 20-55°C, comme les stearyl dimethicones (notamment DC2503 et DC25514 de Dow Corning) ; et leurs mélanges.

Le ou les corps gras pâteux peuvent être présents à raison de 0 à 60% en poids, par rapport au poids total de la composition, de préférence à raison de 0,1 à 45% en poids et encore plus préférentiellement à raison de 2 à 30% en poids, dans la composition, s'ils sont présents.

La composition de l'invention peut, en outre contenir comme additif complémentaire une phase aqueuse contenant de l'eau, éventuellement épaissie ou gélifiée par un épaississant ou un gélifiant de phase aqueuse, et éventuellement des composés miscibles à l'eau. La phase aqueuse peut représenter de 0 à 60% en poids, et notamment de 0,5 à 60% en poids du poids total de la composition.

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

La composition selon l'invention peut se présenter sous la forme d'une composition de soin teintée ou non pour matières kératiniques comme la peau, les lèvres et/ou les phanères, ou sous forme d'une composition d'hygiène corporelle notamment sous forme de produit déodorant ou démaquillant sous forme de stick,
ou sous forme d'un produit de maquillage des lèvres, appelé "top coat", à appliquer sur le rouge à lèvres en "bicouche" pour éviter qu'il ne migre. Elle peut notamment être utilisée comme base de soin pour la peau, les phanères ou les lèvres (baumes à lèvres, protégeant les lèvres du froid et/ou du soleil et/ou du vent, crème de soin pour la peau, les ongles ou les cheveux).

Avantageusement, la composition contient au moins une matière colorante.

La matière colorante selon l'invention peut être choisie parmi les colorants lipophiles, les colorants hydrophiles, les pigments et les nacres habituellement utilisés dans les compositions cosmétiques ou dermatologiques, et leurs mélanges. Cette matière colorante est généralement présente à raison de 0,01 à 90 % du poids total de la composition, de préférence de 0,5 à 60 % et mieux de 2 à 30 %, si elle est présente. Dans le cas d'une composition sous forme de poudre libre ou compactée, la quantité de matière colorante sous forme de particules solides non solubles dans le milieu (nacres et/ou pigments) peut aller jusqu'à 90 % du poids total de la composition, alors qu'elle va jusqu'à 60%

pour les autres formes galéniques.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C orange 5, le jaune quinoléine, le rocou. Ils peuvent représenter de 0,1 à 20 % du poids de la composition et mieux de 0,1 à 6 % (si présents). Les colorants hydrosolubles sont par exemple le jus de betterave, le bleu de méthylène, et peuvent représenter jusqu'à 6 % du poids total de la composition.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium. Les pigments peuvent représenter de 0,1 à 50 %, de préférence de 0,5 à 40 % mieux de 2 à 30 % du poids total de la composition, s'ils sont présents.

Les pigments nacrés peuvent être choisis parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth ou encore les pigments interférentiels ou gogniochromatiques. Ils peuvent représenter de 0,1 à 30 % du poids total de la composition et mieux de 0,1 à 20 %, s'ils sont présents et par exemple de 1 à 15%.

La composition de l'invention peut également se présenter sous la forme d'un produit de maquillage, en particulier coloré, de la peau, en particulier un fond de teint, présentant éventuellement des propriétés de soin, un blush, un fard à joues ou à paupières, un produit anti-cerne, un eye-liner ; un produit de maquillage des lèvres comme un rouge à lèvres, présentant éventuellement des propriétés de soin, un brillant à lèvres, les crayons à lèvres ; un produit de maquillage des phanères comme les ongles, les cils en particulier sous forme d'un mascara pain, les sourcils et les cheveux sous forme de crayon.

Bien entendu la composition de l'invention doit être cosmétiquement ou dermatologiquement acceptable, à savoir contenir un milieu physiologiquement acceptable non toxique et susceptible d'être appliquée sur la peau, les phanères ou les lèvres d'êtres humains.

Par cosmétiquement acceptable, on entend au sens de l'invention une composition d'aspect, d'odeur, de goût et de toucher agréables.

L'invention est illustrée plus en détail dans les exemples suivants. Les quantités sont données en pourcentage massique.

## EXEMPLES

### Exemples comparatifs $E_1$ à $E_4$

On a préparé un premier cas de composition témoin $E_1$ en mélangeant du palmitate de 2-éthylhexyle et du LGB. La composition témoin $E_1$ ne contient pas d'additif polaire.

On a également préparé trois cas de composition selon l'invention $E_2$ à $E_4$ en mélangeant du palmitate de 2-éthylhexyle, du LGB et de l'octyldodécanol comme additif polaire.

Les compositions $E_1$ à $E_4$ sont présentées dans le tableau 1 ci-après.

TABLEAU 1

| Compositions | Octyldodécanol (additif polaire) | LGB | Palmitate de 2-éthylhexyle |
|---|---|---|---|
| $E_1$ (témoin) | 0% | 2% | qsp 100% |
| $E_2$ | 5% | 2% | qsp 100% |
| $E_3$ | 10% | 2% | qsp 100% |
| $E_4$ | 20% | 2% | qsp 100% |

Pour l'ensemble des cas de composition $E_1$ à $E_4$, le mélange du palmitate de 2-éthylhexyle, du LGB et de l'octyldodécanol (le cas échéant) se fait sous agitation à température ambiante. Le mélange est chauffé jusqu'à obtention d'une phase fluide homogène transparente (la température nécessaire pour cette homogénéisation varie entre 90 et 150°C en fonction des proportions d'octyldodécanol). On laisse le mélange homogène et fluide refroidir jusqu'à température ambiante. On obtient alors des compositions solides et dures, qui ne s'affaissent pas à température ambiante

On a mesuré le module complexe $G^* = (G'^2+G''^2)^{1/2}$ qui traduit la consistance des produits à 25° C, exprimé en Pa ainsi que l'angle de perte ou déphasage δ qui traduit le caractère solide ou visqueux du produit.

On a utilisé un rhéomètre à contrainte imposée, Haake RS 150 (société Rhéo), avec un corps de mesures cône-plan de diamètre 35 mm et d'angle 2° dont les surfaces sont sablées pour s'affranchir des problèmes de glissement. Toutes les mesures ont été réalisées à 25 °C.

Pour les paramètres obtenus avant cisaillement, chaque échantillon est soumis à une série de contraintes sinusoïdales de fréquence fixe (1 Hz) et d'amplitudes croissantes réparties de façon logarithmique entre deux extrémités à raison de 5 contraintes par décade.

Les échantillons cisaillés sont soumis pendant 30 s à une vitesse de cisaillement de 1000 $s^{-1}$. A la suite, les aptidudes à la restructuration des échantillons sont appréhendées par le suivie au cours du temps des paramètres G* et δ définis ci-dessous. Pour cela, les échantillons sont soumis à une série de contraintes sinusoïdales de fréquence fixe (1 Hz) d'amplitude constante située dans le domaine viscoélastique linéaire.

Les paramètres rhéologiques mesurés, sont les suivants :

- le module de conservation ou module élastique G',
- le module de perte ou module visqueux G",
- le module complexe G*,
- l'angle de perte ou le déphasage δ, avec tan(δ) = G"/G' ; plus le matériau est élastique, plus δ se rapproche de 0° et plus il est visqueux, plus δ se rapproche de 90°.

Les paramètres G* et δ avant et après cisaillement sont rassemblés dans le tableau 2.

TABLEAU 2

| Composition | δ avant cisaillement (en °) | δ après cisaillement et 10 mn de repos (en °) | G* (Pa) avant cisaillement | G* après cisaillement et t min de repos (en °) | |
|---|---|---|---|---|---|
| | | | | t =1 | t=10 |
| $E_1$ | 7,5 | 9 | 1,3x10$^5$ | 1080 | 2660 |
| $E_2$ | 7,7 | 5 | 1,7x10$^5$ | 3580 | 20800 |
| $E_3$ | 7,6 | 3 | 1,5x10$^5$ | 7200 | 29700 |
| $E_4$ | 7,7 | 5,6 | 1,4x10$^5$ | 3970 | 24700 |

Les compositions $E_1$ à $E_4$ se présentent sous forme solide, avec une forte consistance avant étalement.

On constate, par ailleurs, une reprise de consistance et de dureté supérieure en présence d'octyldodécanol ($E_2$ à $E_4$) par rapport à la composition témoin $E_1$ sans octyldodécanol.

Par ailleurs, le caractère solide défini par l'angle de perte est maintenu avant et après cisaillement. Plus δ est faible et plus la composition a un caractère solide. Les différences de mesure indiquées dans le tableau ne sont pas significatives.

On constate, en outre, grâce à des tests sensoriels que les compositions selon l'invention ($E_2$ à $E_4$) sont moins migrantes que la composition témoin $E_1$ exempte d'octyldodécanol.

**<u>Exemple de composition 1</u>**

**Fond de teint coulé**

- GP-1 d'Ajinomoto 2 %
- Octyldodécanol 8 %
- Pigments ( oxyde de fer jaune, brun) 10 %
- Poudres de Nylon ® 3 %
- Parfum 0,2 %
- BHT 0,07 %
- Palmitate de 2-éthylhexyle qsp 100 %

## Revendications

1. Utilisation d'au moins un additif polaire ayant un paramètre de polarité $\delta_a$ égal ou supérieur à 7,0 $(J/cm^3)^{1/2}$, dans une composition cosmétique comprenant une phase grasse liquide contenant au moins une huile apolaire ou faiblement polaire ayant un paramètre de polarité $\delta_a$ inférieur à 7,0 $(J/cm^3)^{1/2}$, structurée par au moins un organogélateur moléculaire, pour conférer un caractère thixotrope à la composition.

2. Utilisation d'au moins un additif polaire ayant un paramètre de polarité $\delta_a$ égal ou supérieur à 7,0 $(J/cm^3)^{1/2}$, dans une composition cosmétique comprenant une phase grasse liquide contenant au moins une huile apolaire ou faiblement polaire ayant un paramètre de polarité δa inférieur à 7,0 $(J/cm^3)^{1/2}$, structurée par au moins un organogélateur moléculaire, comme agent pour limiter, voire supprimer la migration de la phase grasse.

3. Utilisation d'au moins un additif polaire ayant un paramètre de polarité $\delta_a$ égal ou supérieur à 7,0 $(J/cm^3)^{1/2}$, dans une composition cosmétique comprenant une phase grasse liquide contenant au moins une huile apolaire ou faiblement polaire ayant un paramètre de polarité $\delta_a$ inférieur à 7,0 $(J/cm^3)^{1/2}$, structurée par au moins un organogélateur moléculaire, comme agent pour limiter, voire supprimer la formation de stries sur les paupières.

4. Utilisation d'au moins un additif polaire ayant un paramètre de polarité $\delta_a$ égal ou supérieur à 7,0 $(J/cm^3)^{1/2}$, dans une composition cosmétique comprenant une phase grasse liquide contenant au moins une huile apolaire ou faiblement polaire ayant un paramètre de polarité $\delta_a$ inférieur à 7,0 $(J/cm^3)^{1/2}$, structurée par au moins un organogélateur moléculaire, pour rendre la composition non fracturante, et/ou favoriser l'étalement de la composition sur la peau et/ou pour former un dépôt sur les matières kératiniques, lisse, homogène, non granuleux, continu.

5. Utilisation selon l'une quelconque des revendications 1 à 4, **caractérisée en ce que** l'additif polaire est un bon donneur ou un bon accepteur de liaisons hydrogènes, choisi parmi les alcools gras, les acides gras, les diols, les esters, les éthers, les pyrrolidones, les cétones, les sulfoxydes, et leurs mélanges.

6. Utilisation selon la revendication 5, **caractérisée en ce que** l'additif est choisi parmi l'octyldodécanol, l'hexyldécanol, l'octyldécanol, l'undécylpentadécanol, l'hexylène glycol, le 1,2-hexanediol, le propylène glycol, la glycérine, l'alcool oléique, l'alcool phényléthylique, l'huile de rincin, l'éthyl-myristate, l'isopropyl-palmitate, le Finsolv, le diisopropyl-sébaçate, le diisopropyl-adipate, le propylène glycol dicaprate/dicaprylate et leurs mélanges.

7. Utilisation selon la revendication 5 ou 6, **caractérisée en ce que** l'additif polaire est présent dans la composition à raison de 1 à 40% en poids, et de préférence de 5 à 30 % en poids, et mieux de 8 à 20% en poids par rapport au poids total de la composition.

8. Utilisation selon l'une quelconque des revendications précédentes, **caractérisée en ce que** l'organogélateur moléculaire est un organogélateur dont les molécules comportent au moins un groupement capable d'établir des liaisons hydrogènes et mieux au moins deux groupements capables de faire des liaisons hydrogènes, au moins un cycle aromatique et mieux au moins deux cycles aromatiques, au moins une ou plusieurs liaisons à insaturation éthylénique et/ou au moins un ou plusieurs carbones asymétriques.

9. Utilisation selon l'une quelconque des revendications précédentes, **caractérisée en ce que** l'organogélateur moléculaire est choisi parmi les carboxyliques hydroxylés à chaîne carbonée aliphatique linéaire ou ramifiée comportant notamment au moins 8 atomes de carbone ; les amides d'acides carboxyliques ; les amides ou esters d'amino acides ; les amides de N-acylamino acides et notamment les diamines résultant de l'action d'un N-acylamino acide avec des amines comportant de 1 à 22 atomes de carbone ; les diamides ayant de 1 à 22 atomes de carbone et mieux de 6 à 18 atomes, éventuellement substitués ; les amines ou amides de stéroïde et leurs sels ; les azobenzène stéroïdes ; les composés organométalliques ; les tensioactifs sous forme de sel comportant au moins deux chaînes alkyles linéaires ou ramifiées ayant de 8 à 30 atomes de carbone ; les benzylidènes sorbitols ou alditols et dérivés ; et leurs mélanges.

10. Utilisation selon l'une quelconque des revendications précédentes, **caractérisée en ce que** l'organogélateur moléculaire est choisi parmi les N-acylamino acides, les amides de N-acylamino acides, les cyclohexane tricarboxamides, les cyclohexane diamines et leursmélanges.

11. Utilisation selon l'une quelconque des revendications précédentes, **caractérisée en ce que** l'organogélateur moléculaire représente de 0,1 à 60% en poids, de préférence de 1 à 30% en poids, et mieux de 2 à 20 % en poids

du poids total de la composition.

**12.** Utilisation selon l'une quelconque des revendications précédentes, **caractérisée en ce que** l'huile apolaire est une huile hydrocarbonée apolaire, choisie parmi l'huile de parléam, le squalane, les hydrocarbures aliphatiques, et leurs mélanges.

**13.** Utilisation selon l'une quelconque des revendications 1 à 11, **caractérisée en ce que** l'huile apolaire est une huile de silicone apolaire choisie parmi les polydiméthylsiloxanes (PDMS) et les silicones phénylées telles que les phényltriméthicones, les phényldiméthicones, les diphényldiméthicones, les diphényl méthyldiphényl trisiloxanes et les 2-phényléthyl triméthylsiloxysilicates, et leurs mélanges.

**14.** Utilisation selon l'une quelconque des revendications 1 à 11, **caractérisée en ce que** les huiles faiblement polaires, ayant un paramètre de polarité $\delta_a$ inférieur à 7,0 $(J/cm^3)^{1/2}$, sont choisies parmi les esters d'acide et/ou d'alcool gras en $C_8$-$C_{70}$, tels que le palmitate d'éthyle hexyle et le trimellitate de tridécyle.

**15.** Utilisation selon l'une quelconque des revendications précédentes, **caractérisée en ce que** la phase grasse contient :

- soit au moins une huile apolaire présente dans la composition à raison de 0,2 à 98,9% en poids, de préférence à raison de 1 à 80% en poids, et mieux de 5 à 60% en poids par rapport au poids de la phase grasse,
- soit au moins une huile faiblement polaire, ayant un paramètre de polarité $\delta_a$ inférieur à 7,0 $(J/cm^3)^{1/2}$, présente dans la composition à raison de 0,2 à 98,9% en poids, de préférence de 1 à 80% en poids, et mieux de 5 à 60% en poids par rapport au poids de la phase grasse.

**16.** Utilisation selon l'une quelconque des revendications précédentes, **caractérisée en ce que** la phase grasse liquide représente de 0,2 à 98,9% en poids, de préférence de 10 à 80% en poids, et mieux de 5 à 60% en poids du poids total de la composition.

**17.** Utilisation selon l'une quelconque des revendications précédentes, **caractérisée en ce que** la phase grasse liquide contient également une ou plusieurs huiles polaires, ayant un paramètre de polarité $\delta_a$ égal ou supérieur à 7,0 $(J/cm^3)^{1/2}$, choisies parmi l'octyldodécanol, l'hexyldécanol, l'octyldécanol, l'undécylpentadécanol, l'hexylène glycol, le 1,2-hexanediol, le propylène glycol, la glycérine, l'alcool oléique, l'alcool phényléthylique, l'huile de ricin, l'éthyl-myristate, l'isopropylpalmitate, le Finsolv, le diisopropyl sébaçate, le diisopropyladipate, le propylène glycol dicaprate/dicaprylate et leurs mélanges.

**18.** Composition cosmétique comprenant au moins une phase grasse liquide contenant au moins une huile hydrocarbonée apolaire ou faiblement polaire ayant un paramètre de polarité $\delta_a$ inférieur à 7,0 $(J/cm^3)^{1/2}$, structurée par au moins un organogélateur moléculaire de ladite phase grasse liquide à l'exclusion de tout gélifiant polymérique de masse moléculaire moyenne en poids allant de 1000 à 100.000 et comportant un squelette polymérique ayant des motifs urée, uréthane, thiourée, thiouréthane et/ou amide, et au moins un additif polaire ayant un paramètre de polarité $\delta_a$ égal ou supérieur à 7,0 $(J/cm^3)^{1/2}$, la phase grasse, l'organogélateur moléculaire et l'additif formant un milieu physiologiquement acceptable.

**19.** Composition selon la revendication 18, **caractérisée en ce que** l'additif est choisi parmi les alcools gras, les acides gras, les diols, les esters, les éthers, les pyrrolidones, les cétones, les sulfoxydes, et leurs mélanges.

**20.** Composition selon la revendication 19, **caractérisée en ce que** l'additif est choisi parmi l'octyldodécanol, l'hexyldécanol, l'octyldécanol, l'undécylpentadécanol, l'hexylène glycol, le 1,2-hexanediol, le propylène glycol, la glycérine, l'alcool oléique l'alcool phényléthylique, l'huile de ricin, l'éthyl-myristate, l'isopropyl-palmitate, le Finsolv, le diisopropyl-sébaçate, le diisopropyl-adipate, le propylène glycol dicaprate/dicaprylate et leurs mélanges.

**21.** Composition selon l'une quelconque des revendications 18 à 20, **caractérisée en ce que** l'organogélateur moléculaire est choisi parmi les acides gras carboxyliques hydroxylés à chaîne carbonée aliphatique linéaire ou ramifiée comportant notamment au moins 8 atomes de carbone ; les amides d'acides carboxyliques ; les amides ou esters d'amino acides ; les amides de N-acylamino acides et notamment les diamides résultant de l'action d'un N-acylamino acide avec des amines comportant de 1 à 22 atomes de carbone ; les diamides ayant de 1 à 22 atomes de carbone et mieux de 6 à 18 atomes, éventuellement substitués ; les amines ou amides de stéroïde; les azobenzène stéroïdes ; les composés organométalliques, les tensioactifs sous forme de sel comportant au moins deux chaînes

alkyles linéaires ou ramifiées ayant de 8 à 30 atomes de carbone ; les benzylidènes sorbitols ou alditols et dérivés ; et leurs mélanges.

**22.** Composition selon la revendication 21, **caractérisée en ce que** l'organogélateur moléculaire est choisi parmi les N-acylamino acides, les amides de N-acylamino acides, les cyclohexane tricarboxamides, les cyclohexane diamines et leurs mélanges.

**23.** Composition selon l'une quelconque des revendications 18 à 22, **caractérisée en ce qu'**elle constitue une composition de soin et/ou de maquillage des matières kératiniques.

**24.** Composition selon l'une quelconque des revendications 18 à 23, **caractérisée en ce qu'**elle contient au moins un additif choisi parmi l'eau, les antioxydants, les huiles essentielles, les conservateurs, les parfums, les charges, les cires, les composés gras pâteux à température ambiante, les neutralisants, les polymères liposolubles ou dispersibles dans le milieu, les actifs cosmétiques ou dermatologiques, les dispersants, et leurs mélanges, l'additif étant présent à raison de 0 à 20% en poids, notamment de 0,01 à 20% en poids, et mieux de 0,01 à 10% en poids du poids total de la composition.

**25.** Composition selon l'une quelconque des revendications 18 à 24, **caractérisée en ce qu'**elle se présente sous forme de stick.

**26.** Composition selon l'une quelconque des revendications 18 à 24, **caractérisée en ce qu'**elle se présente sous forme de produit déodorant.

**27.** Composition selon l'une quelconque des revendications 18 à 24, **caractérisée en ce qu'**elle se présente sous forme de produit de maquillage des lèvres à appliquer sur le rouge à lèvres en bicouche.

**28.** Composition selon l'une quelconque des revendications 18 à 27, **caractérisée en ce qu'**elle contient au moins une matière colorante choisie parmi les colorants lipophiles, les colorants hydrophiles, les pigments, les nacres et leurs mélanges, la matière colorante étant présente à raison de 0,01 à 50% en poids, et de préférence de 0,5 à 40% en poids par rapport au poids total de la composition.

**29.** Composition selon la revendication 28, **caractérisée en ce qu'**elle se présente sous forme d'un rouge à lèvres.

Office européen des brevets

# RAPPORT DE RECHERCHE EUROPEENNE

Numéro de la demande
EP 02 29 1423

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | WO 00 15180 A (HERCULES INC) 23 mars 2000 (2000-03-23) * le document en entier * | 1-7, 12-14, 16,17, 19,20, 23,28 | A61K7/027 A61K7/00 A61K7/48 |
| X | WO 98 55081 A (PROCTER & GAMBLE) 10 décembre 1998 (1998-12-10) * page 19, alinéas 5-5 - page 20, alinéa 1; revendications; exemples * | 1-9, 12-14, 16,17, 19,20, 23,28 | |
| X | WO 98 55095 A (PROCTER & GAMBLE) 10 décembre 1998 (1998-12-10) * page 11, alinéa 2 * * page 12, alinéa 2 * | 1-9, 12-14, 16,17, 19,20, 23,28 | DOMAINES TECHNIQUES RECHERCHES (Int.Cl.7) A61K |
| A | EP 0 795 322 B (OREAL) 17 septembre 1997 (1997-09-17) * revendications * | 1 | |

Le présent rapport a été établi pour toutes les revendications

| Lieu de la recherche | Date d'achèvement de la recherche | Examinateur |
|---|---|---|
| LA HAYE | 11 septembre 2002 | Couckuyt, P |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE RELATIF A LA DEMANDE DE BREVET EUROPEEN NO.** EP 02 29 1423

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

11-09-2002

| Document brevet cité au rapport de recherche | Date de publication | Membre(s) de la famille de brevet(s) | Date de publication |
|---|---|---|---|
| WO 0015180 A | 23-03-2000 | AU 6041499 A | 03-04-2000 |
| | | BR 9913617 A | 22-05-2001 |
| | | CN 1316896 T | 10-10-2001 |
| | | EP 1112054 A1 | 04-07-2001 |
| | | WO 0015180 A1 | 23-03-2000 |
| WO 9855081 A | 10-12-1998 | US 6287577 B1 | 11-09-2001 |
| | | AU 745392 B2 | 21-03-2002 |
| | | AU 7704898 A | 21-12-1998 |
| | | BR 9811706 A | 25-07-2000 |
| | | CN 1265028 T | 30-08-2000 |
| | | EP 0996420 A2 | 03-05-2000 |
| | | JP 2001518941 T | 16-10-2001 |
| | | WO 9855081 A2 | 10-12-1998 |
| | | US 6217887 B1 | 17-04-2001 |
| | | US 6294186 B1 | 25-09-2001 |
| | | ZA 9804765 A | 04-12-1998 |
| WO 9855095 A | 10-12-1998 | US 6214363 B1 | 10-04-2001 |
| | | AU 7704698 A | 21-12-1998 |
| | | CN 1264293 T | 23-08-2000 |
| | | EP 0996419 A1 | 03-05-2000 |
| | | JP 2002501542 T | 15-01-2002 |
| | | WO 9855095 A1 | 10-12-1998 |
| | | US 6294186 B1 | 25-09-2001 |
| | | ZA 9804768 A | 04-12-1998 |
| EP 0795322 B | 17-09-1997 | FR 2746107 A1 | 19-09-1997 |
| | | CA 2198922 A1 | 13-09-1997 |
| | | DE 69700041 D1 | 03-12-1998 |
| | | DE 69700041 T2 | 25-03-1999 |
| | | EP 0795322 A1 | 17-09-1997 |
| | | ES 2126408 T3 | 16-03-1999 |
| | | JP 2983487 B2 | 29-11-1999 |
| | | JP 10001444 A | 06-01-1998 |

EPO FORM P0460

Pour tout renseignement concernant cette annexe : voir Journal Officiel de l'Office européen des brevets, No.12/82